# EUROPEAN PATENT APPLICATION

(11) **EP 3 069 679 A1**
(43) Date of publication of application: **21.09.2016**
(21) Application number: 15159575.8
(22) Date of filing: 18.03.2015
(51) Int. Cl.: A61B 34/20, G02B 27/01, A61B 17/00

(54) **A system for precision guidance of surgical procedures on a patient**

(71) Applicant: Metronor AS, 1360 Nesbru (NO)
(72) Inventor: Røtvold, Øyvind, 1395 Hvalstad (NO)
(74) Representative: Lang, Johannes

(57) **Abstract**

A system for precision guidance of surgical procedures on a patient in order to position an object precisely relative to a movable patient. The system comprising a measurement means for measuring the position and orientation of a relevant part of a patient whereto a first object such as an implant or surgical instrument is to be brought into an intended, pre-defined spatial relation, projection means providing a life-size virtual but observable 3D image of said first object, means for determining the relationship between the coordinate system of the measurement means and the coordinate system of the projection means, thereby enabling the virtual but observable 3D image of said first object to be projected in such a way as to represent the intended position and orientation of the first object relative to the relevant part of the patient.

## Description

Surgical guidance systems - often referred to as Computer Assisted Surgery or CAS systems - are systems that assist a surgeon in positioning - both with respect to position and orientation - one or more objects relative to the relevant parts of a patient undergoing surgery. Examples of objects to be navigated include biopsy needles, implants, surgical templates, pins and screws or surgical instruments. Navigation implies that an intended or desired position for the object is known, and that the navigation system provides - preferable real-time and continuous - feedback on the difference between this object's current and intended positions, thus enabling the surgeon to minimize this difference and thereby position the object in precisely the intended position.

Surgical guidance systems can also be used to position two objects relative to each other, such as placing an instrument in contact with an implant or simultaneously position two objects relative to the patient, such as e.g. a transmitter and a receiver.

Such surgical navigation systems are known. To be truly useful and interfere as little as possible with the surgical procedure, such systems need to be fairly complex from a measurement point-of-view. The main challenge is that the patient will move about during surgery, and therefore the intended position of the object to be navigated will also be moving relative to any fixed measurement system or reference. This challenge is exacerbated by the fact that the measurement device should also be moveable in order to not interfere unduly with the surgical team's activities.

A common problem with surgical guidance systems is communicating clearly and intuitively to the surgeon how to move the object in order to attain the intended position and orientation. In current solutions, this information is provided on a conventional computer display. Prior art recognizes this problem and therefore includes provisions intended to partially mitigate the difficulty, such as to automatically orient the display to match the viewing point of the surgeon. Other methods, such as object perspective views, multiple simultaneous displays along several axes, axis slider views and axis handles are also known.

The key difficulty is how to provide an immediate and intuitive understanding to the surgeon of the needed movement of the object based on the surgeon watching a flat 2D display of the three dimensional problem.

The problem to be overcome in the surgical application actually has two main components:
(1) How to show the difference between the position and orientation of two three-dimensional bodies - in our case, one representing the current actual object position and the other representing the nominal or intended object position in such a way that it is instantly and intuitively understandable to humans.
(2) Looking away from the patient and onto a two-dimensional display reduces the surgeon's attention on the patient and is ergonomically unfortunate. In cases where the surgeon wears loupes or other optical aids during surgery, this problem is obviously exacerbated. Ideally, the guidance information should be available to the surgeon without removing attention from the patient.

This problem is solved according to the invention by a system according to independent claims 1 and 10. Advantageous embodiments of the inventive system are defined in the dependent claims.

Methods for displaying virtual, but observable ₃D object images are known *per se* from the prior art. Such methods include holographic projection, as well as various ways to present virtual, but observable ₃D object images onto user-worn glasses, goggles or other transparent or semi-transparent displays in such a way that the ₃D images appear to the wearer to be part of the physical scene in front of him/her.

Such methods therefore promise a means of providing a live, intuitively understandable three-dimensional image of the nominal or intended object position to the user.

Note, however, that it is necessary that such a virtual, but observable ₃D object image appears to the user to be fixed relative to the patient regardless of patient and/or user movement during the procedure. Therefore, an integrated solution as defined in the independent claims is needed that can (1) track the patient position, (2) track the user position, (3) use this information to calculate continuously updated information on the nominal position and orientation, where the virtual object is to be displayed, and (4) display the virtual object so it appears patient-fixed to the user.

The system according to the present invention overcomes both of the challenges facing current surgical guidance solutions - the surgeon does not need to remove his attention from the patient, and the intended object position is shown as a virtual object inside the physical world negating the need to interpret any two-dimensional representations. The positioning process becomes intuitive, and full focus can be retained on the surgical process itself. The benefits of Computer Assisted Surgery therefore becomes available without the current penalty of decreased surgeon focus on the patient procedure and increased training requirements.

### Embodiments

In practical applications, two distinct types of implementations are considered: (1) Embodiments using a projection system to create the virtual, but observable ₃D object image, and (2) embodiments using a user-worn display as glasses or goggles to create the virtual, but observable 3D object image.

### Projection system

When using a projection system to create the virtual, but observable 3D object image, the relationship between the projection system and the user is not critical, as the projection system will create a virtual object in space observable from a range of orientations. However, as the patient is likely to move during surgery, it is critical that the exact position of the patient is continuously tracked and that this information is fed back to the projection system, so that the position and orientation of the virtual, but observable ₃D object image is updated to match. This will ensure that the nominal intended position represented by the virtual, but observable ₃D object image appears patient-fixed to the user.

To achieve this, the following problems must be overcome:
(1) The coordinate system of the projection system must be aligned with the coordinate system of the measurement system tracking the patient movement, and
(2) the position and orientation of the virtual, but observable 3D object must be made to coincide with the nominal intended object position, and
(3) the position and orientation of the virtual, but observable ₃D object must be continuously updated as the patient moves.

Clearly, it is necessary to adjust the projection system in such a way that the size, position and orientation of the virtual projected object is well-defined and well-controlled inside the coordinate system of the projection system. This adjustment, which is internal to the projection system, should of course be completed before aligning coordinate systems of the projection system and measurement system.

The alignment of coordinate systems need not be physical; the requirement is to determine the relationship between the two coordinate systems - e.g. in the form of a transformation matrix, or a set of points known in both coordinate systems - so that any information known in the coordinate system of e.g. the measurement system can be determined also in the coordinate system of the projection system.

One way to achieve this alignment is to position a physical object into the working volume of the projection system and the measurement system, and to then use a joystick or similar interface to precisely adjust the position and orientation of the virtual, but observable ₃D object image of the same object, so that the physical and the virtual objects match precisely. The virtual object will now be known in the projection system's internal coordinate system. The physical object can be determined in the coordinate system of the measurement system by simply measuring the position of some characterizing features - e.g. the corners of a cube or other polyhedron - and by knowing multiple locations in both coordinate systems the relationship between the two can be determined.

As a similar practical approach, a number of virtual ₃D spheres can be projected into a volume, and a sphere-shaped target or probe of matching size can then be physically moved about to sequentially overlay or match each of the virtual spheres precisely, thus yielding a series of center-points known both in the projection and the measurement system's coordinate systems.

The precision of the alignment will improve if the locations of the points or characterizing features known in both coordinate systems are separated widely inside the system's intended working volume. By ensuring that the locations used to determine the alignment covers a majority of the working volume, the precision of the alignment will be dramatically improved over a local alignment restricted to a small sub-volume - the improvement in precision is largely proportional to the increase in the longest distance between locations used.

In order to update the projection placement in response to any patient movements, the prior art teaches that attaching to the patient an object with a set of targets in known, relative positions would enable a measurement camera to continuously determine the patient position. Since the previous steps means that the patient position can then be calculated also in the projection system coordinate system, a corresponding updated position for the virtual ₃D object can also be calculated.

It should be realized that for operations where very minute object movements corresponding to extremely precise object positioning is required, it may be advantageous to augment the virtual, but observable ₃D object image with additional indications of remaining movement. Examples of such augmentations could be a change in virtual ₃D object color, or the 3D display itself might include continuously updated 3D arrows exaggerating the remaining movement.

To facilitate such an augmented display, it will be helpful to attach a set of targets in known, relative positions also on the object being positioned, thus augmenting the user's visual impression of remaining movement with mathematical deviation data generated by the system computer based on the deviation between the actual and intended position and orientation of said object.

### User-worn display

For the embodiment utilizing a user-worn display, the system becomes more complex as the spatial relationship between three units needs to be continuously determined or tracked for the solution to work as intended: (1) The patient, (2) the user wearing the display, as goggles or glasses, and (3) the measurement camera. It is easily realized that since the intended position of the virtual ₃D object needs to appear stationary relative to the patient as seen through the user's display, as goggles or glasses, then any user movement relative to the patient will need to result in a corresponding movement of the display inside the user's display, as goggles or glasses.

It is noticed that if the measurement camera is placed in a fixed position relative to one of the other units, e.g. integrated into the user's display, goggles or glasses, then the relative movement of the user and the patient is the only spatial relationship that needs to be tracked.

Also notice that if an augmented display for very minute object movements corresponding to extremely precise object positioning is required, it may be advantageous to augment the virtual ₃D object image with additional indications of remaining movement in the same way as was outlined for the projection system embodiment above. In such a case, it will be helpful to attach a set of targets in known relative positions also on the physical object being positioned, thus augmenting the user's visual impression of remaining movement with mathematical deviation data generated by the system computer based on the deviation between the actual and intended position and orientation of said object.

## Claims

1. A system for precision guidance of surgical procedures on a patient, said system comprising
a) a measurement means for measuring the position and orientation of a relevant part of a patient whereto a first object such as an implant or surgical instrument is to be brought into an intended, pre-defined spatial relation,
b) projection means providing a life-size virtual but observable ₃D image of said first object,
c) means for determining the relationship between the coordinate system of the measurement means and the coordinate system of the projection means, thereby enabling the virtual but observable ₃D image of said first object to be projected in such a way as to represent the intended position and orientation of the first object relative to the relevant part of the patient.

2. The system according to claim 1, wherein
the measuring means comprises
a) an electro-optical camera,
b) a first carrier comprising at least 3 targets to be observed by said camera, said carrier mounted in a known position and orientation relative to the relevant part of the patient,
c) software for calculating positions and orientations of the relevant part of the patient from camera images of the targets.

3. The system according to claims 1 or 2, wherein
the means for determining the relationship between the coordinate system of the measurement means and the coordinate system of the projection means comprises
a) a second object, to which is mounted in a known relationship a further carrier comprising at least 3 targets to be observed by the camera,
b) software for determining the relationship between the coordinate system of the projection means and the coordinate system of the measurement means by (1) placing the second object into the system's working volume, (2) registering the second objects' position and orientation by the measuring means, and (3) using a joystick or similar input device to guide the projecting means until a virtual ₃D image of the second object is aligned with the physical second object.

4. The system according to claim 2, wherein
the means for determining the relationship between the coordinate system of the measurement means and the coordinate system of the projection means comprises
a) a further carrier comprising at least 3 targets to be observed by the camera, said carrier having a stylus that can be brought into contact with points on a further object in order to determine the coordinates of the points in the coordinate system of the measurement means,
b) software for determining the relationship between the coordinate system of the projection means and the coordinate system of the measurement means by (1) projecting a virtual but observable ₃D image of any object into the system's working volume, (2) placing one or more additional physical objects matching at least three geometrical features of the virtual but observable ₃D image of said any object using the stylus of the further carrier to register the location of these features in the coordinate system of the measuring means.

5. The system according to any one of claims 1 to 4, wherein the projection means is a holographic projection system.

6. The system according to any one of claims 1 to 4, wherein the projection means is a user-wearable display, as goggles or glasses providing a virtual but observable ₃D image of the first object, said display having attached to it in a known relationship a further carrier comprising at least 3 targets to be observed by the camera.

7. The system according to any one of claims 1 to 4, wherein the projection means is a user-wearable display, as goggles or glasses providing a virtual but observable ₃D image of the first object, said display having attached to it in a known relationship the camera of the measurement means.

8. The system according to any one of claims 2 to 7, wherein
a) a further carrier comprising at least 3 targets to be observed by the camera, is attached in a known relationship to the first object,
b) the software calculates the difference between the position of the first object and the intended position of the first object, and
c) the projection means displays indications of the movement needed to align the first object with its' intended position as part of the virtual but observable ₃D image of said first object.

9. The system according to claim 8, wherein there are at least two further carriers each comprising at least 3 targets to be observed by the camera , each further carrier to be mounted in a known position and orientation relative to one of at least two separate first objects.

10. A system for precision guidance of surgical procedures on a patient, said system comprising
a) a three-dimensional digital representation of the intended position of a first object to be attached or brought into contact with a relevant part of the patient,
b) measuring means comprising
- an electro-optical camera for measurement,
- a first carrier comprising at least three targets to be observed by the camera, said carrier mounted in a known position and orientation relative to the relevant part of the patient,
c) a computer with software for determining the relative positions of the first carrier and of the camera, based on observations of the targets by the camera,
**characterized by**
d) means for creating a virtual, but observable ₃D image of the first object and
e) means for aligning the coordinate system of the measuring means and the coordinate system of the means for creating a virtual, but observable ₃D image of the first object, in order to position said virtual, but observable 3D image of the first object in the intended position of the first object relative to the relevant part of the patient.

11. The system according to claim 10, wherein
the means for creating a virtual, but observable ₃D image of the first object are projecting means, and
the aligning means comprise at least one third carrier comprising at least three targets to be observed by the camera, said third carrier mounted in a known position and orientation relative to a second object with distinct characterizing features, whereby the mathematical transformation between the coordinate system of the aligning means and the coordinate system of the projecting means is determined by placing the at least one third carrier and the associated second object in the working volume of the system and whereby a joystick or a similar input device is used to guide the projecting means until a projected virtual, but observable ₃D image of the second object is aligned with the second object and whereby, based on this transformation and the current observations of the first carrier, the computer software calculates the relevant position and orientation in the coordinate system of the projecting means where the projecting means shall create a virtual, but observable ₃D image of the intended position and orientation of the first object thus providing visual guidance to the user.

12. The system according to claim 10, wherein
the means for creating a virtual, but observable ₃D image of the first object are projecting means and
the aligning means comprise a third carrier, comprising at least three targets to be observed by the camera, said third carrier mounted in a known position and orientation relative to a stylus for indicating points for which the coordinates are to be determined using the measuring means, whereby the mathematical transformation between the coordinate system of the aligning means and the coordinate system of the projecting means is determined by projecting a virtual, but observable ₃D image of at least three third objects inside the working volume of the system and measuring the position of the virtual, but observable 3D image of the at least three third objects by (1) aligning respectively a physical object to each of the said virtual, but observable ₃D image of the at least three third objects and (2) measuring the position of the at least three physical objects with a stylus of the third carrier,
whereby,
based on this mathematical transformation and current observations of the first carrier, the computer software calculates the relevant position and orientation in the coordinate system of the projecting means, where the virtual, but observable ₃D image of the first object is to be positioned to provide visual guidance for the user.

13. The system according to claim 10, wherein
the means for creating a virtual, but observable ₃D image of the first object is a user-wearable display such as goggles or glasses and
the aligning means comprise
a third carrier comprising at least three targets to be observed by the camera, whereby the third carrier is attached to the user-wearable display, whereby the mathematical transformation between the coordinate system of the virtual space into which the user-wearable display can display a virtual, but observable ₃D image and
the coordinate system of the measuring means is determined by
(1) fixedly mounting the user-wearable display,
(2) determining the positions of the at least three targets of the third carrier using the measuring means,
(3) projecting a virtual, but observable ₃D image of at least three second objects inside the working volume of the system and
(4) measuring the position of the at least three second objects in the coordinate system of the measuring means by (i) aligning respectively a physical object to each of the virtual, but observable ₃D image of the at least three second objects and (ii) measuring the position of the at least three physical objects with a stylus of a fourth carrier, said fourth carrier comprising at least three targets to be observed by the camera and a stylus for identifying points to be measured,
whereby,
based on this mathematical transformation and current observations of the first carrier and third carrier, the computer software calculates the relevant position and orientation in the coordinate system of the virtual space into which the user-wearable display can display a virtual, but observable ₃D image, where the virtual, but observable ₃D image of the first object is to be positioned to provide visual guidance for the user.

14. The system according to any one of claims 1 to 9, wherein the camera is a digital camera calibrated to precisely measure spatial directions to targets in space.

15. The system according to any one of claims 1 to 13, wherein the targets are active light emitting elements.
